# EUROPÄISCHE PATENTSCHRIFT

(11) **EP 0 412 465 B1**
(45) Veröffentlichungstag und Bekanntmachung des Hinweises auf die Patenterteilung: **28.09.1994**
(21) Anmeldenummer: 90115004.5
(22) Anmeldetag: 04.08.1990
(51) Int. Cl.: C07K 3/08

(54) **Verfahren zur biokatalytischen korrekten Kettenfaltung denaturierter rekombinanter Fusionsproteine**
Process for correct biocatalytic chain folding of denaturated recombinant fusion proteins
Procédé pour le pliage correct biocatalytique des chames de protéines de fusion recombinantes dénaturées

(30) Priorität: 08.08.1989 DE 3926103
(43) Veröffentlichungstag der Anmeldung: 13.02.1991
(73) Patentinhaber: HOECHST AKTIENGESELLSCHAFT, 65926 Frankfurt am Main (DE)
(72) Erfinder: Neupert, Walter, Prof. Dr. Dr., D-8034 Germering (DE); Hartl, Franz-Ulrich, Dr., D-8044 Unterschleissheim (DE)

(56) Entgegenhaltungen:
- CELL, Band 46, 26. September 1986, Seiten 959-961, Cell Press; H.R.B. PELHAM: "Speculations on the functions of the major heat shock and glucose-regulated proteins"
- BIOLOGICAL ABSTRACTS, Band 88, Nr. 4, 1989, Zusammenfassung Nr. 36801, Biological Abstracts, Inc., Philadelphia, PA, US; S.J. LANDRY et al.: "The small subunit of ribulose-1, 5-bisphosphate catboxylase/oxygenase and its precursor expressed in Escherichia coli are associated with groEL protein"
- BIOLOGICAL ABSTRACTS, Band 90, Nr. 6, 1990, Zusammenfassung Nr. 59769, Biological Abstracts, Inc., Philadelphia, PA, US; W.P. SHEFFIELD et al.:"Mitochondrial precursor protein: Effect of 76-kilodalton heat shock protein on polypeptide folding, aggregation and import competence"
- NATURE, Band 344, Nr. 6269, 26. April 1990, Seiten 882-884, Macmillan Magazines Ltd, London, GB; G.J. PHILLIPS et al.: "Heat-shock proteins DnaK and GroEL facilitate export of LacZ hybrid proteins in E. coli"

## Beschreibung

Die vollständige Information für die im funktionell aktiven Zustand eines Proteins vorliegende Kettenfaltung ist in der Aminosäuresequenz des Proteins enthalten. Diese Aussage ist dadurch praktisch belegt, daß - wenn auch nur in ausgewählten Fällen - die korrekte Rückfaltung artifiziell entfalteter Proteine in vitro demonstriert werden konnte (Anfinsen, C.B., Science 181: 223-230, 1973). Der Prozess der Proteinfaltung, insbesondere komplexer Proteine mit multiplen Domänen oder mehreren Untereinheiten, wird jedoch nur sehr lückenhaft verstanden (Jänicke, R., Biophys. Struct. Mech. 8: 231-256 1982), und in den letzten Jahren wurden hier keine wesentlichen Fortschritte erzielt. Die Untersuchung der Proteinfaltung in vivo wurde völlig vernachlässigt, da angenommen wurde, daß prinzipiell auch unter physiologischen Bedingungen Proteine in einem spontanen "Self Assembly"-Prozess falten (Creighton, T., in "Proteins", Freeman und Company, New York, 1984). Diese Auffassung ist jedoch mit größter Wahrscheinlichkeit falsch. Viele Proteine, die nach Synthese in vivo in Sekunden bis wenigen Minuten ihr funktionelle Konformation erreichen, lassen sich nach Auffaltung unter verschiedenen denaturierenden Bedingungen in vitro nicht rückfalten. In letzter Zeit häufen sich Befunde, di zu der Hypothese führten, daß die Konformation von Proteinketten physiologischerweise durch spezielle Komponenten, im wesentlichen Hitzeschockproteine, beeinflußt wird (Pelham, H., Nature 332: 776-777, 1988). Hitzeschockproteine sind Proteine, welche in den Zellen von Organismen vorhanden sind und in vermehrtem Umfang gebildet werden, wenn die Zellen besonderen Belastungen wie z.B. Hitzebelastungen unterworfen werden.

Es wurde bereits eine Proteinkomponente identifiziert und funktionell charakterisiert, die in vivo und in vitro die korrekte Kettenfaltung entfalteter bzw. neusynthetisierter Polypeptide katalysiert. Es handelt sich um das in Bakterien und Mitochondrien vorkommende Hitzeschockprotein GroEL bzw. Hsp60. Die Aminosäuresequenzen von GroEL in Escherichia coli und Hsp60 in Mitochondrien zeigen 60 % Identität (Hemmingsen, S.M. Woolford, C., van der Vies, S.M. Tilly, K., Dennis, D.T., Georgopoulos, C.P. Hendrix, R.W. und Ellis, R.J., Nature 333: 330-334, 1988; Reading, D.S. Hallberg, R.L., Myers, A.M., Nature 337: 655-659, 1989). Die Komponenten liegen als praktisch identische oligomere Komplexe aus 14 Untereinheiten mit Molekulargewichten von je etwa 58 000 Dalton vor. Gemäß der Erfindung binden künstlich entfaltete oder naszierende Proteine an die Oberfläche des GroEL- bzw. Hsp60-Komplexes. Dabei besteht keine Spezifität für bakterielle oder mitochondriale Proteine. Die erfindungsgemäße Kettenfaltung vollzieht sich an GroEL bzw. Hsp60 in einer von ATP (= Adenosintriphosphat)-Hydrolyse abhängigen Reaktion. Die Beteiligung einer weiteren mit dem GroEL- bzw. Hsp60-Komplex assoziierten Komponente an diesem Prozeß ist nicht auszuschließen. In Abwesenheit von ATP bindet z.B. das Enzym Dihydrofolatreduktase der Maus als entfaltete, hoch Protease-sensitive Polypeptidkette an Hsp60. ATP-Zugabe führt zu einer wesentlichen Zunahme der zunächst noch an der Oberfläche von Hsp60 assoziierten Dihydrofolatreduktase mit anschließender Freisetzung des Enzyms, das dann in seiner enzymatisch aktiven Konformation vorliegt. Diese Reaktion kann im Innenraum der Mitochondrien in einer Proteinkonzentration von etwa 30 bis 50 g Protein/100 ml ablaufen. Sie ist jedoch in prinzipiell gleicher Weise mit isoliertem Hsp60 in vitro möglich. Die Ausschaltung der Hsp60-Funktion in einer konditionalen Hefemutante führt zum Ausfall der Assemblierung mitochondrialer Proteine zu supramolekularen Komplexen (Cheng, M.Y., Hartl, F.-U., Martin, J., Pollock, R.A., Kalousek, F., Neupert, W., Hallberg, E.M., Hallberg, R.L., und Horwich, A.L., Nature 337: 620-625, 1989).

GroEl und Hsp60 sind löslich, relativ unempfindlich gegen unspezifische Proteasen wie Proteinase K und leicht in erheblichen Mengen aus E. coli und dergleichen zu isolieren. Eine Isolierungsmethode für GroEl ist z.B. beschrieben in der Literaturstelle Hendrix, R.W., J. Mol. Biol. 129: 375-392 (1979); GroEL ist dort als gp groE bezeichnet.

Im Falle der Isolation von Hsp60 aus Mitochondrien liefert ein in 0.3 % Digitonin, 100 mM NaCl, 30 mM Tris (= Tris-(hydroxymethyl)aminomethan) bei pH 7.4 hergestellter Extrakt in zwei Reinigungsschritten durch Ammonsulfatfällung (35 bis 50 % Sättigung) und Q-^{(R)}Sepharose-Chromatographie das Protein Hsp60 in einer guten Ausbeute und einer ausreichenden Reinheit; Q-Sepharose ist ein basischer Ionenaustauscher auf Agarose-Basis der Fa. Pharmacia LKB GmbH, Freiburg (Bundesrepublik Deutschland).

Es wurde nun gefunden, daß sich auch denaturierte rekombinante Fusionsproteine in Gegenwart von Hitzeschockproteinen, welche nach dem funktionellen Prinzip des bakteriellen GroEL oder des äquivalenten mitochondrialen Hsp60 wirken, korrekt falten lassen; die Hitzeschockproteine haben hier die Funktion von Biokatalysatoren.

Erfindungsgegenstand ist daher ein Verfahren zur biokatalytischen korrekten Kettenfaltung denaturierter rekombinanter Fusionsproteine,
das dadurch gekennzeichnet ist,
daß man eine wäßrige Probe enthaltend denaturierte rekombinante Fusionsproteine, wobei der Fusionsanteil mindestens eine Aminosäure enthält, bei einem pH-Wert von etwa 6 bis 8 mit einem Hitzeschockprotein, welches die gleiche katalytische Wirkung bei der Kettenfaltung hat wie GroEl oder Hsp60 und Adenosintriphosphat in Kontakt bringt.

In Fusionsproteinen tragen die Aminosäuresequenzen der chimären Partner keine Information, um gegenseitig die Faltung zur nativen Struktur der Einzelkomponenten zu induzieren. Deshalb ist es sehr überraschend, daß Fusionsproteine, die häufig aus einem mikrobiellen und einem eukaryotischen Protein zusammengesetzt sind, mit einem entsprechenden Hitzeschockprotein in ein Protein gefaltet werden, dessen unterschiedliche chimäre Partner in ihrer natürlichen Struktur kovalent gebunden sind. Der eigentlich interessierende Proteinanteil kann hieraus dann durch spezifische Proteolyse oder - wenn vorher eine säurelabile Spaltstelle eingebaut wurde - durch saure Hydrolyse freigesetzt werden.

Auf diese Weise können Proteine von biologischem und medizinischem Interesse auf vorteilhafte und relativ einfache Weise in der richtigen - funktionell aktiven - Konformation gewonnen werden.

Rekombinante Fusionsproteine werden durch gentechnologische Methoden hergestellt. Der Fusionsanteil muß mindestens eine Aminosäure enthalten. Die Obergrenze der Zahl der Aminosäurereste im Fusionsanteil kann in einem weiten Bereich schwanken; vorzugsweise liegt sie bei etwa 400.

Die gentechnologische Herstellung von Peptiden und Proteinen durch Expression der klonierten Gene in Bakterien (in der Regel in Form einer Fusion an Fremdsequenzen, wie β-Galactosidase) resultiert häufig in der Produktion sogenannter Einschlußkörperchen (inclusion bodies), in denen das entsprechende Fusionsprotein als unlösliches, funktionell inaktives Aggregat vorliegt. Aus dieser Zustandsform läßt sich das (Fusions-)Protein in der Regel nur durch denaturierende Bedingungen in eine lösliche Form überführen. Dabei wird das (Fusions)-Protein ganz oder zumindest teilweise artifiziell entfaltet. In etwas weiterem Sinn werden hier unter denaturierten rekombinanten Fusionsproteinen un- oder mißgefaltete sowie ganz oder teilweise artifiziell entfaltete Fusionsproteine verstanden.

Die Denaturierung der gentechnologisch erhaltenen - zunächst unlöslichen, funktionell inaktiven - rekombinanten Fusionsproteinen erfolgt nach üblichen Methoden durch Einwirkung denaturierender Agenzien in wäßriger Lösung, z.B. mittels 6 bis 8 M Harnstoff in einem Tris- oder Glycin-Puffer bei pH etwa 6 bis 9. Falls das Fusionsprotein Disulfidbrücken enthält, ist zur Entfaltung (auch) ein SH-Gruppen enthaltendes Reagenz, wie z.B. Mercaptoethanol oder Dithiothreitol erforderlich und zwar in einer Menge entsprechend mindestens etwa 2 SH-Gruppen pro Disulfidbrücke.

Der Denaturierungsansatz (mit dem nun entsprechend entfalteten und gelösten Fusionsprotein) wird dann in der Regel verdünnt, so daß die Fusionsprotein-Konzentration vorzugsweise bei etwa 0.01 bis 0.5 mg/ml (bestimmt nach Lowry) liegt; im speziellen Einzelfall können u.U. auch höhere Konzentrationen (bis zu etwa 100 mg/ml) angebracht bzw. möglich sein.

Die Erfindung beginnt nun mit dem Zusatz eines entsprechenden Hitzeschockproteins und von ATP.

Als Hitzeschockproteine kommen im Prinzip alle nach dem funktionellen Prinzip des bakteriellen GroEL oder des äquivalenten mitochondrialen Hsp60 wirkenden Hitzeschockproteine in Frage; bevorzugt sind GroEL und Hsp60, insbesondere Hsp60. Natürlich kann auch mehr als ein Hitzeschockprotein gleichzeitig eingesetzt werden, was im allgemeinen aber keine Vorteile erbringt.

Weiterhin ist es auch nicht erforderlich, das jeweilige Hitzeschockprotein in reiner Form einzusetzen; es genügt vielmehr auch der Einsatz in Form eines angereicherten Extrakts.

Das molare Verhältnis von zu faltendem Fusionsprotein und dem Biokatalysator für die korrekte Kettenfaltung (Hitzeschockprotein) kann in weiten Grenzen schwanken; bevorzugt ist ein Verhältnis von etwa (10 bis 100):1.

Die Konzentration des ATP (welches bevorzugt als Mg²⁺-ATP verwendet wird) kann ebenfalls in relativ weiten Grenzen schwanken; bevorzugt liegt sie bei etwa 1 bis 50 mM, insbesondere bei etwa 2 bis 10 mM.

Weniger breit ist dagegen der pH-Bereich für die erfindungsgemäße Biokatalyse; er liegt bei etwa 6 bis 8. Der pH-Wert wird auf übliche Weise, z.B. mittels eines Tris- oder Glycin-Puffers in etwa 10 bis 150 mM Konzentration eingestellt, wobei auch die Anwesenheit von etwa 50 bis 200 mM NaCl oder KCl vorteilhaft ist.

Die Temperatur für die korrekte Kettenfaltung liegt in der Regel zwischen etwa 15 und 30°C.

Nicht zu hohe Konzentrationen von Denaturierungsreagenzien - von der Denaturierungsreaktion her - wie z.B. bis zu etwa 0,8 M Harnstoff oder bis zu etwa 0.6 M Guanidiniumhydrochlorid stören für die korrekte Faltung im allgemeinen nicht.

Der Faltungsansatz wird dann auf übliche Weise aufgearbeitet und das Fusionsprotein mit der richtigen Faltung der beiden chimären Bestandteile zur Gewinnung des gewünschten Proteins in der Regel proteolytisch oder acidolytisch gespalten.

Die Erfindung wird nun durch die folgenden Beispiele näher erläutert.

### Beispiele

a) Fusionsprotein Dihydrofolatreduktase-Fo-ATPase.
   Ein durch rekombinante Gentechnologie hergestelltes Fusionsprotein zwischen Dihydrofolatreduktase der Maus und der aminoterminalen Sequenz (Reste 1-66) der Untereinheit 9 der mitochondrialen FoF₁-ATPase wird in 6 M Harnstoff, 30 mM Tris-HCl, pH 7.4, denaturiert (Proteinkonzentration 0.3 mg/ml).
   Kontrollreaktion: nach Zugabe von 10 »l dieser Lösung zu 290 »l 2 mM ATP, 2mM MgCl₂, 100 mM NaCl, 30 mM Tris-HCl, pH 7.4 und Inkubation für 20 min bei 25°C bleibt der Dihydrofolatreduktase-Teil des Proteins unvollständig gefaltet und erreicht nicht die enzymatisch aktive Konformation.
   Zugabe von 10 »g/ml isoliertem Hsp60 zum gleichen Ansatz erzeugt die enzymatisch aktive Konformation der Dihydrofolatreduktase nach Inkubation für 5 min bei 25°C mit einer Ausbeute von 50 - 70 %. Die Ausbeute wird über die spezifische Aktivität des nach der Reaktion als lösliches Monomer vorliegenden Fusionsproteins (gereinigt durch Gelfiltration über ^{(R)}Superose 6, d.i. ein Molekularsieb aus quervernetzter Agarose der Fa. Pharmacia LKB GmbH, Freiburg) gemessen und beträgt 92 % der nativer Dihydrofolatreduktase. Nach Abspaltung der Fremdsequenz durch die spezifische Prozessierungspeptidase der Mitochondrien wird die volle spezifische Aktivtät erreicht (s. Beispiel b).
b) Authentische Dihydrofolatreduktase (Vergleich):
   Wie Ansatz (a), aber Verwendung von authentischer Dihydrofolatreduktase als Substrat. Die Ausbeute des in der enzymatisch aktiven Konformation vorliegenden Proteins (98 % der erreichbaren spezifischen Aktivität) beträgt 60 - 80 %. Dihydrofolatreduktase ohne durch rekombinante Gentechnologie angekoppelte Fremdsequenzen zeigt eine gewisse Tendenz zur spontanen Rückfaltung. Im Kontrollansatz betrug die Ausbeute an korrekt gefaltetem Enzym unter den verwendeten Versuchsbedingungen aber nur 30 %. (Dihydrofolatreduktase enthält keine S-S-Brücken).
c) Fusionsprotein Interleukin-2-β-Galaktosidase:
   Ein in E. coli exprimiertes Fusionsprotein zwischen Interleukin-2 und den 400 aminoterminalen Resten der E.coli-β-Galaktosidase wird aus Einschlußkörperchen bei einer Proteinkonzentration von 0.2 mg/ml in 8M Harnstoff/50 mM Glycinpuffer, pH 7.2 gelöst. Entsprechend der Anzahl der SH-Gruppen werden molare Äquivalente an Mercaptoethanol zugegeben. Das gelöste Fusionsprotein (100 »g) wird jeweils in 20 »g Portionen 10-fach in einen Ansatz verdünnt, der 5 »g/ml Hsp60 in 50 mM Glycinpuffer, pH 7.2 enthält. Die Faltungsreaktion wird durch Zugabe von 2 mM Mg²⁺-ATP gestartet und für 15 min bei 25°C ausgeführt. Anschließend wird unter Spaltung einer säurelabilen Bindung (-Asparaginsäure-Prolin-) das Interleukin-2 aus dem Fusionsprotein freigesetzt und durch präparative HPLC-Trennung gereinigt. Die Retentionszeit des in vitro gefalteten Produkts war mit der eines Interleukin-2 Standards identisch. Ebenso entsprachen sich die Fragmentmuster des Faltungsprodukts und des Standards nach Trypsinproteolyse. Die spezifische Aktivität, bestimmt durch Zytotoxizitätstest an CTLJK-Zellen (= cytotoxische Lymphzellen mit der Bezeichnung JK) betrug 9800 U/mg Protein (Standard 10000 U/mg; Proteinbestimmung nach Lowry). Die Ausbeute der Faltungsreaktion betrug 58 %. Aus der Kontrollreaktion ohne Zugabe von Hsp60 konnte kein Interleukin-2 freigesetzt werden.

## Patentansprüche

1. Verfahren zur biokatalytischen korrekten Kettenfaltung denaturierter rekombinanter Fusionsproteine, dadurch gekennzeichnet, daß man eine wäßrige Probe, enthaltend denaturierte rekombinante Fusionsproteine, wobei der Fusionsanteil mindestens eine Aminosäure enthält, bei einem pH-Wert von etwa 6 bis 8 mit einem Hitzeschockprotein, welches die gleiche katalytische Wirkung bei der Kettenfaltung hat wie GroEl oder Hsp60 und Adenosintriphosphat in Kontakt bringt.

2. Verfahren nach Anspruch 1, dadurch gekennzeichnet, daß man die Konzentration des Ausgangs-Fusionsproteins auf etwa 0.01 bis 0.5 mg/ml einstellt.

3. Verfahren nach Anspruch 1 oder 2, dadurch gekennzeichnet, daß man als Hitzeschockprotein GroEL und/oder Hsp60, insbesondere nur Hsp60, verwendet.

4. Verfahren nach einem oder mehreren der Ansprüche 1 bis 3, dadurch gekennzeichnet, daß man das Hitzeschockprotein in Form eines angereicherten Extrakts verwendet.

5. Verfahren nach einem oder mehreren der Ansprüche 1 bis 4, dadurch gekennzeichnet, daß man das molare Verhältnis von Fusionsprotein zu Hitzeschockprotein auf etwa (10 bis 100):1 einstellt.

6. Verfahren nach einem oder mehreren der Ansprüche 1 bis 5, dadurch gekennzeichnet, daß man Adenosintriphosphat in einer Konzentration von etwa 1 bis 50 mM, vorzugsweise von etwa 2 bis 10 mM, einsetzt.

7. Verfahren nach einem oder mehreren der Ansprüche 1 bis 6, dadurch gekennzeichnet, daß man die korrekte Kettenfaltung bei einem pH-Wert von etwa 6 bis 8 durchführt.

8. Verfahren nach einem oder mehreren der Ansprüche 1 bis 7, dadurch gekennzeichnet, daß man die korrekte Kettenfaltung bei einer Temperatur im Bereich von etwa 15 bis 30°C durchführt.

9. Verwendung von Hitzeschockproteinen, welche die gleiche katalytische Wirkung bei der Kettenfaltung wie GroEl oder Hsp60 haben, als Biokatalysatoren für die korrekte Kettenfaltung denaturierter rekombinanter Fusionsproteine, wobei der Fusionsanteil mindestens eine Aminosäure enthält.

10. Verwendung nach Anspruch 9, dadurch gekennzeichnet, daß die Hitzeschockproteine GroEL und/oder Hsp60, insbesondere nur Hsp60, sind.

## Claims

1. A process for the biocatalytic, correct chain folding of denatured recombinant fusion proteins, which comprises bringing an aqueous sample comprising denatured recombinant fusion proteins, where the fused portion contains at least one amino acid, into contact at a pH of about 6 to 8 with a heat shock protein which has the same catalytic effect in chain folding as GroEl or Hsp60 and adenosine triphosphate.

2. The process as claimed in claim 1, wherein the concentration of the initial fusion protein is adjusted to about 0.01 to 0.5 mg/ml.

3. The process as claimed in claim 1 or 2, wherein GroEl and/or Hsp60, in particular only Hsp60, is used as heat shock protein.

4. The process as claimed in one or more of claims 1 to 3, wherein the heat shock protein is used in the form of an enriched extract.

5. The process as claimed in one or more of claims 1 to 4, wherein the molar ratio of fusion protein to heat shock protein is adjusted to about (10 to 100):1.

6. The process as claimed in one or more of claims 1 to 5, wherein adenosine triphosphate is used in a concentration of about 1 to 50 mM, preferably of about 2 to 10 mM.

7. The process as claimed in one or more of claims 1 to 6, wherein the correct chain folding is carried out at a pH of about 6 to 8.

8. The process as claimed in one or more of claims 1 to 7, wherein the correct chain folding is carried out at a temperature in the range from about 15 to 30°C.

9. The use of heat shock proteins which have the same catalytic effect in chain folding as GroEl or Hsp60 as biocatalysts for the correct chain folding of denatured recombinant fusion proteins, where the fused portion contains at least one amino acid.

10. The use as claimed in claim 9, wherein the heat shock proteins are GroEL and/or Hsp60, in particular only Hsp60.

## Revendications

1. Procédé pour le repliement correct biocatalytique de protéines de fusion recombinantes dénaturées, caractérisé en ce que l'on met en contact un échantillon aqueux, contenant des protéines de fusion recombinantes dénaturées, la fraction fusionnée contenant au moins un aminoacide, à un pH d'environ 6 à 8, avec une protéine de choc thermique qui a le même effet catalytique dans le repliement de chaîne que GroEL ou Hsp60, et de l'adénosine triphosphate.

2. Procédé selon la revendication 1, caractérisé en ce que l'on ajuste la concentration de la protéine de fusion de départ aux environs de 0,01 à 0,5mg/ml.

3. Procédé selon la revendication 1 ou 2, caractérisé en ce que, en tant que protéine de choc thermique, on utilise GroEL et/ou Hsp60, en particulier seulement Hsp60.

4. Procédé selon une ou plusieurs des revendications 1 à 3, caractérisé en ce que l'on utilise la protéine de choc thermique sous forme d'un extrait concentré.

5. Procédé selon une ou plusieurs des revendications 1 à 4, caractérisé en ce que l'on ajuste le rapport molaire de la protéine de fusion à la protéine de choc thermique aux environs de 10:1 à 100:1.

6. Procédé selon une ou plusieurs des revendications 1 à 5, caractérisé en ce que l'on utilise de l'adénosine triphosphate à une concentration d'environ 1 à 50 mM, de préférence d'environ 2 à 10 mM.

7. Procédé selon une ou plusieurs des revendications 1 à 6, caractérisé en ce que l'on effectue le repliement correct de la chaîne à un pH d'environ 6 à 8.

8. Procédé selon une ou plusieurs des revendications 1 à 7, caractérisé en ce que l'on effectue le repliement correct de la chaîne à une température dans la plage allant d'environ 15 à 30°C.

9. Utilisation de protéines de choc thermique qui ont le même effet catalytique dans le repliement de chaîne que GroEL ou Hsp60, en tant que biocatalyseurs pour le repliement correct de chaînes de protéines de fusion recombinantes dénaturées, la fraction fusionnée contenant au moins un aminoacide.

10. Utilisation selon la revendication 9, caractérisée en ce que les protéines de choc thermique sont GroEL et/ou Hsp60, en particulier seulement Hsp60.
